Europäisches Patentamt

European Patent Office

Office européen des brevets

(11) Publication number: **0 516 132 A1**

# EUROPEAN PATENT APPLICATION

(21) Application number: **92109069.2**

(22) Date of filing: **29.05.92**

(51) Int. Cl.5: **A61K 31/70**

(30) Priority: **31.05.91 IT PD910101**

(43) Date of publication of application:
**02.12.92 Bulletin 92/49**

(84) Designated Contracting States:
**AT BE CH DE DK ES FR GB GR IT LI LU MC NL PT SE**

(71) Applicant: **FIDIA S.p.A.**
**Via Ponte della Fabbrica 3-A**
**I-35031 Abano Terme (Padova)(IT)**

(72) Inventor: **Romeo, Aurelio**
**Viale Ippocrate 93**
**I-00161 Rome(IT)**
Inventor: **Leon, Alberta**
**Piazza Napoli 19**
**Padova(IT)**

(74) Representative: **Vossius & Partner**
**Siebertstrasse 4 P.O. Box 86 07 67**
**W-8000 München 86(DE)**

(54) Anti-neuronotoxic activity and therapeutic applications of GM1 ganglioside methyl ester.

(57) Provided is a new use of the methyl ester of $GM_1$ ganglioside for preventing or treating neuronotoxic damage in a human or animal subject.

Such damage can be associated with ischemia, hypoxia, trauma and compression, metabolic dysfunction, epilepsy, neurodegenerative disease, chronic pain, and cerebral infarct. Also provided is a pharmaceutical composition, comprising the methyl ester of $GM_1$ ganglioside and a pharmaceutically acceptable excipient, vector, or diluent.

EP 0 516 132 A1

The present invention relates to a new therapeutic use for the methyl ester of the ganglioside $GM_1$ in preventing and/or treating neuronotoxic damage.

The carboxylic esters of gangliosides are described in European patent application No. 85401291.1 filed on June 5, 1985, with Italian priority dated July 3, 1984. Their therapeutic use, like that of the gangliosides themselves, is based on their ability to stimulate "sprouting" of nerve cells, thereby stimulating recovery of nervous conduction. This "neurotrophic" effect can be attributed to incorporation of the ganglioside derivatives in the neuronal membrane, which induces a modification of their functional properties, i.e., of their enzymatic properties. In particular, incorporation of said derivatives can stimulate $(Na^+,K^+)$-ATPase activity. Some authors ascribe neuronal survival and consequent differentiation in culture to activation of this enzyme by NGF (Varon S., Skaper S. D.: The $Na^+,K^+$ pump may mediate the control of nerve cells by NGF. TIBS 8, 22-25, 1983). On the other hand, this enzyme's key role in electric activity is well known, being involved in the ionic mechanism underlying propagation of the nervous impulses along the axonal membranes.

Owing to the above properties, ganglioside esters, which have a longer-lasting neuronotrophic effect than basic gangliosides, can be used as drugs in various therapies for the treatment of nervous system pathologies, in particular those affecting the peripheral and central nervous systems.

They can be employed in therapies for peripheral nervous system pathologies of a compressive traumatic, degenerative, metabolic or toxic-infective origin, in which it is necessary to stimulate nervous regeneration and recovery of neuromuscular function, and in pathologies affecting the central nervous system of traumatic, anoxic, degenerative or toxic-infective origin, in which it is necessary to stimulate neuronal sprouting phenomena for functional recovery.

The present invention concerns the use of the methyl ester of the ganglioside $GM_1$ in preventing and/or treating neuronotoxic damage in humans and animals. This ester has, apart from the properties noted above, characteristic of all gangliosides and their esters, a specific and marked anti-neurotoxic effect which can be exploited to counteract damage to the nervous system caused by the action of excitatory amino acids, such as in the case of ischemia, hypoxia, trauma and compression, metabolic dysfunction, epilepsy, in chronic neurodegenerative diseases (J. W. Olney: Excitotoxic amino acids and neuropsychiatric disorders. Ann. Rev. Pharmacol. Toxicol. 30, 47-71, 1990), in chronic pain (Sugimoto T. et al.: Transsynaptic degeneration in the superficial dorsal horn after sciatic nerve injury: effects of a chronic constriction injury, transection and strychnine. Pain, 42, 205-213, 1990; Woolf C. J. et al.: The induction and maintenance of central sensitization is dependent on N-methyl-D-aspartic acid receptor activation: implications for the treatment of post-injury pain hypersensitivity states. Pain, 44, 293-299, 1991), and in some pathologies affecting the neurovegetative system (Glavin G.B. et al.: Kinurenic acid attenuates experimental ulcer formation and basal gastric secretion in rats. Res. Com. in Chem. Path. and Pharm. 64, 1, 111-119, 1989). This anti-neuronotoxic action can be effective both in preventing said damage and in repairing it.

Neuronal damage, in particular acute cerebral damage, can be of various origins, but apart from its etiological causes, its outcome is secondary neurodegeneration mediated by over-activation of excitatory amino acid (EAA) receptors. This activation unleashes a cascade of cellular events (activation of $Ca^{+2}$-dependent enzymes, $Ca^{+2}$ influx, second messenger activation) which results in neuronal death. The damaged neurons may die during and/or immediately after damage (primary neuronal death) or later (secondary neuronal death). Surviving neurons are capable of undergoing reparative neuroplastic phenomena, the expression of which is regulated by neuronotrophic factors (NTF). Therefore, while molecules capable of limiting and/or preventing neurotoxicity by EAAS are active in providing protection from acute damage, molecules capable of modulating trophic factors can limit degenerative phenomena over a longer period. Both of these properties are found in gangliosides and their derivatives. For example, the monosialoganglioside $GM_1$ can limit EAA-induced neurotoxicity (anti-neuronotoxic effect) at an early stage, and can modulate NTF activity (pro-neuronotrophic effect) over a longer period (reviewed in: Skaper S. D.: Ganglioside function in the development and repair of the nervous system. Molecular Neurobiology 3, 173-199, 1989). In particular, it has been reported that gangliosides in vitro can limit EAA-induced neurotoxicity (Favaron M. et al.: Gangliosides prevent glutamate and kainate neurotoxicity in primary neuronal cultures of neo-natal rat cerebellum and cortex. Proc. Natl. Acad. Sci. USA: 85: 7351-7355, 1988; Skaper S. D. et al.: Gangliosides attenuate the delayed neurotoxicity of aspartic acid in vitro. Neurosci. Lett. 117, 154-159, 1990). In parallel, the acute, neuroprotective effects of the monosialoganglioside $GM_1$ have been extensively described in the literature, in in vivo experimental models of global and focal cerebral ischemia (Komatsumoto S. et al.: Effect of the ganglioside $GM_1$ on neurologic function, electroencephalogram amplitude and histology in chronic middle cerebral artery occlusion in cats. Stroke, 19: 1027-1035; reviewed in: Karpiak S. E. et al. Ganglioside reduction of CNS ischemic injury. CRC Critical Rev. in Neurobiology. Vol. 5 Issue 3, 1990;) and in models of acute neurotoxicity induced by excitatory amino acids (Lipartiti M. et

al.: Monosialoganglioside $GM_1$ reduces NMDA neurotoxicity in neonatal rat brain. Proceedings of the 17th Princetown Conference on Cerebrovascular Disease, March 16-18, 1990). On the other hand, it has been reported that $GM_1$ diminishes secondary neurodegenerative effects and facilitates functional recovery, after not only ischemic, but also traumatic, insult (Toffano G. et al.: Chronic $GM_1$ ganglioside treatment reduces dopamine cell body degeneration in the substantia nigra after unilateral hemitransection in the rat. Brain Res.; 296: 233-239, 1984) or toxic insult (Lombardi G. et al.: Systemic treatments with $GM_1$ ganglioside reduce quinolinic acid-induced striatal lesions in the rat. Eur. J. Phamacol. 174: 123-125, 1989; Mahadik S. P. et al.: $GM_1$ ganglioside acutely reduces excitotoxin damage in nucleus basalis: cortical cholinergic losses and mortality. J. Neurosci. Res. 20: 479-483, 1989; Schneider J. S. et al.: $GM_1$ ganglioside treatment promotes recovery of striatal dopamine concentrations in the mouse model of MPTP-induced parkinsonism. Exp. Neurol. 105: 177-183, 1989).

Although no data are to be found in the literature concerning the anti-neuronotoxic activity of the methyl ester of the $GM_1$ ganglioside, one would reasonably have assumed that it would be comparable to that of the basic ganglioside $GM_1$. Unexpectedly, however, as demonstrated by the experimental results presented infra, the anti-neuronotoxic activity of the methyl ester of $GM_1$ is very pronounced and is significantly greater than that of the $GM_1$ ganglioside.

This result is particularly striking if one considers that esterification of the $GM_1$ ganglioside with methyl alcohol does not bring about a similar rise in neuronotrophic affect. Esterification of the $GM_1$ ganglioside with said alcohol therefore separates the neuronotrophic effect from the anti-neuronotoxic effect, with the latter predominating.

The data presented infra demonstrate the protective effect in vivo of the methyl ester of ganglioside $GM_1$ on cerebral damage induced by i.c.v. injection of NMDA in newborn rats.

Accordingly, it is an object of the present invention to provide a method for preventing or treating neuronotoxic damage in a subject, comprising administering to said subject an anti-neuronotoxic damage effective amount of the methyl ester of $GM_1$ ganglioside.

It is another object of the present invention to provide a pharmaceutical composition for the prevention or treatment of neuronotoxic damage in a subject, comprising the methyl ester of $GM_1$ ganglioside and a pharmaceutically acceptable excipient, vector, or diluent.

Further scope of the applicability of the present invention will become apparent form the detailed description provided below. However, it should be understood that the detailed description and specific examples, while indicating preferred embodiments of the invention, are given by way of illustration only, since various changes and modifications within the spirit and scope of the invention will become apparent to those skilled in the art from this detailed description.

The following detailed description is provided to aid those skilled in the art in practicing the present invention. Even so, the following detailed description should not be construed to unduly limit the present invention, as modifications and variations in the embodiments herein discussed may be made by those of ordinary skill in the art without departing from the spirit or scope off the present inventive discovery.

The contents of each of the references cited in the present application are herein incorporated by reference in their entirety.

EXAMPLE 1

Materials and Methods

Description of the model

The experiments described herein were conducted on 7-day-old rats weighing about 13 grams.

On the seventh day of life, the animals were anaesthetized with ice and injected i.c.v. with 25 nmole/l $\mu$l of NMDA (N-methyl-D-aspartate, Sigma, St. Louis, MO, USA) according to the method described by McDonald et al. (McDonald J. W. et al.: Neurotoxicity of N-methyl-D-aspartate is markedly enhanced in developing rat central nervous system. Brain Res. 459: 200-203, 1988).

The excitotoxin was solubilized in saline, the pH being brought to a value of 7.4 by the addition of 1N NaOH.

NMDA was injected slowly (over a period of 2 minutes) into the right lateral ventricle using a Hamilton syringe with a stainless steel, 30-gauge needle. Control animals received saline (1 $\mu$l i.c.v.).

Test products: (Solubilization and relative administration):

The following products were tested:
- Monosialoganglioside $GM_1$
- The methyl ester of monosialoganglioside $GM_1$ ($AGF_4$).

The products were solubilized in saline and administered subcutaneously (s.c.) at increasing doses (1-2.5-10-20 mg/kg) to assess the dose-effect of the test products.

The treatment consisted of a two-fold administration::
- 1 hour before injection of NMDA
- immediately after injection of NMDA

Experimental groups

| 1st (n = 9) | saline (1 $\mu$l) + saline (1 ml) |
|---|---|
| 2nd (n = 7) | NMDA (25 nmole/1 $\mu$l) + saline (1 ml) |
| 3rd (n = 8) | NMDA (25 nmole/1 $\mu$l) + $AGF_4$ (1 mg/kg/ml) |
| 4th (n = 8) | NMDA (25 nmole/1 $\mu$l) + $AGF_4$ (2.5mg/kg/ml) |
| 5th (n = 8) | NMDA (25 nmole/1 $\mu$l) + $AGF_4$ (10 mg/kg/ml) |
| 6th (n = 8) | NMDA (25 nmole/1 $\mu$l) + $AGF_4$ (20 mg/kg/ml) |
| 7th (n = 8) | NMDA (25 nmole/1 $\mu$l) + $GM_1$ (1 mg/kg/ml) |
| 8th (n = 8) | NMDA (25 nmole/1 $\mu$l) + $GM_1$ (2.5 mg/kg/ml) |
| 9th (n = 8) | NMDA (25 nmole/1 $\mu$l) + $GM_1$ (10 mg/kg/ml) |
| 10th (n = 8) | NMDA (25 nmole/1 $\mu$l) + $GM_1$ (20 mg/kg/ml) |

The number of animals employed in each experimental group is indicated in brackets as 'n'.

Parameters

The animals were sacrificed on their twelfth day of life (5 days after injection of NMDA) to assess total brain weight, expressed in mg.

Statistical significance was assessed by the Duncan test.

RESULTS

The results obtained (Table 1) from evaluation of the dose-effect of $AGF_4$ versus $GM_1$ (1-2.5-10-20 mg/kg s.c.) indicate that:
- injection of NMDA induces a decrease in total brain weight;
- treatment with $AGF_4$, starting from a dose of 10 mg/kg s.c., significantly reduces ($p<0.05$) cerebral damage induced by the excitotoxin (assessed on the basis of the decrease in total brain weight). $AGF_4$ reaches maximum efficacy ($p<0.01$) at a dose of 20 mg/kg s.c.
- the effect of $AGF_4$ is greater than that of $GM_1$. Indeed, $AGF_4$ is significantly effective at as low a dose as 10 mg/kg s.c., while $GM_1$ is ineffective at the same dose.

4

Table 1

| Anti-neuronotoxic effect of AGF$_4$ (vs GM$_1$) in vivo: assessment of the protection from cerebral damage (total brain weight in mg) induced by NMDA in newborn rats | | | |
|---|---|---|---|
| Groups | Products | dose (mg/kg) | total brain weight (mg) |
| 1st (n = 9) | control + saline | | 1044.7 |
| 2nd (n = 7) | NMDA + saline | | 894.0 |
| 3rd (n = 8) | NMDA + AGF$_4$ | 1 | 890.0 |
| 4th (n = 8) | NMDA + AGF$_4$ | 2.5 | 895.3 |
| 5th (n = 8) | NMDA + AGF$_4$ | 10 | 960.1 * |
| 6th (n = 8) | NMDA + AGF$_4$ | 20 | 1008.3 ** |
| 7th (n = 8) | NMDA + GM$_1$ | 1 | 860.1 |
| 8th (n = 8) | NMDA + GM$_1$ | 2.5 | 890.1 |
| 9th (n = 8) | NMDA + GM$_1$ | 10 | 891.3 |
| 10th (n = 8) | NMDA + GM$_1$ | 20 | 994.3 ** |

\* $p < 0.05$ vs rats with lesions and treated with saline (group 2)
\** $p < 0.01$ vs rats with lesions and treated with saline (group 2) (In all groups, the standard deviation was less than 5%)

The test products were administered at a dose of 1-2.5-10-20 mg/kg S.c. 1 hour before injection of NMDA (25 nmole/1 $\mu$l) and immediately after NMDA injection. Brain weight is expressed in mg.

CONCLUSIONS

The results of the in vivo experiments described above demonstrate the significant anti-neuronotoxic activity of the methyl ester derivative of the monosialoganglioside GM$_1$ (AGF$_4$), making it possible to outline a particular pharmacological profile of the product which differs from that of GM$_1$ or its inner ester, and from the reports in the literature about such products.

Regarding the pharmacological activity of AGF$_4$, it should be emphasized that:
- the two-fold administration of 20 mg/kg s.c. markedly protects rats from cerebral damage induced by i.c.v. injection of NMDA (N-methyl-D-aspartate);
- the anti-neuronotoxic effect of AGF$_4$ is greater than that of GM$_1$. Indeed, AGF$_4$ is significantly efficient at as low a dose as 10 mg/kg s.c., unlike GM$_1$ which is inactive at this dose, and which therefore requires administration at a higher dose (20 mg/kg s.c.) in order to protect against neuronal damage.

A particular profile can therefore be envisaged for the methyl ester of GM$_1$, due to its specific anti-neuronotoxic effect, manifested by the protection it affords from EAA-induced acute cerebral damage: AGF$_4$ is more effective than GM$_1$ in limiting the evolution of primary cerebral damage.

The aforesaid anti-neuronotoxic activity of the methyl derivative of GM$_1$ can be considered predictive of its pharmacological application in nervous system pathologies in which it is necessary to protect against EAA-induced neurotoxicity. Said excitatory amino acids include glutamic acid, aspartic acid, N-methyl-D-aspartic acid, $\beta$-N-oxalylamino-L-alanine, alanosine, cysteic acid, cysteine sulfinic acid, homocysteic acid, cysteine-S-sulfonic acid, ibotenic acid, quisqualic acid, kainic acid, domoic acid, and $\beta$-N-methylamino-L-alanine.

EXAMPLE 2

THERAPEUTIC APPLICATIONS AND PHARMACEUTICAL PREPARATIONS

The therapeutic use of this derivative can therefore be envisaged in all those pathologies in which there is excitotoxin-induced neuronal damage, such as in ischemia, hypoxia, trauma and compression, epilepsy, metabolic dysfunction, neurodegenerative disorders (J. W. Olney: Excitotoxic amino acids and neuropsychiatric disorders. Ann. Rev. Pharmacol. Toxicol. 30:47-71, 1990) and chronic pain (Sugimoto T. et al.: Transsynaptic degeneration in the superficial dorsal horn after sciatic nerve injury: effects of a chronic constriction injury, transection and strychnine. Pain, 42, 205-213, 1990; Woolf C. J. et al.: The induction and maintenance of central sensitization is dependent on N-methyl-D-aspartic acid receptor activation: implications for the treatment of post-injury pain hypersensitivity states. Pain, 44, 293-299, 1991).

By virtue of its anti-neuronotoxic properties, the methyl ester of the present invention can be employed in therapies for various nervous system disorders, such as those reported previously, especially in cases of cerebral infarct. It can be administered as suitable pharmaceutical preparations containing the methyl enter of the $GM_1$ ganglioside at the desired dose, in combination with one or more pharmaceutically acceptable excipients, vectors, or diluents. These pharmaceutical preparations can be administered to humans or animals intramuscularly, subcutaneously, or intradermally, by intravenous injection, or by infusion. The pharmaceutical preparations can be in the form of buffered solutions of the active compound at a suitable pH, osmotically compatible with the physiological fluids. The dose to be administered will depend on the desired effects and on the chosen route of administration. The pharmaceutical preparation can be in the form of an ampule containing the active derivative in solution, possibly with other auxiliary ingredients. For parenteral administration in therapy or as a preventive measure, the dose can be varied preferably between 0.05 mg and 10 mg of active substance per kg of body weight/day, and more preferably between 0.05 mg and 2 mg per kg of body weight/day.

Also encompassed in the present invention are pharmaceutical preparations containing the active ganglioside derivative of the present invention. These can be in solid or semi-solid form, for example pills, tablets, gelatin capsules, capsules, suppositories, and soft gelatin capsules. For parenteral use, those forms intended for intramuscular, subcutaneous or intradermal administration can be used, or those forms suitable for infusion or intravenous injection can be employed. These can therefore be solutions of the active compound which are suitable for the aforesaid uses. For local use, preparations can be in the form of sprays, for example nose sprays, creams, or ointments for topical use, or suitably treated sticking plasters for intradermal administration. Preparations for parenteral use preferably contain between 0.1% and 10% of active compound based on the weight of the total composition. In the case of solutions, sprays, ointments and creams, between 1% and 100% of the active compound can be present, based on the weight of the total composition. Between 5% and 50% of active compound can be present in preparations in solid form, based on the weight of the total composition.

The methyl ester of $GM_1$ ganglioside is itself already known, and can be prepared in any conventional manner. For example, preparative methods have been described in European patent application 85401291.1. Chemical synthesis is illustrated in Example 3, below.

## EXAMPLE 3

### Preparation of the methyl ester of $GM_1$ ganglioside

5g of the inner ester of $GM_1$ ganglioside (3.27 mM) are dissolved in 200 ml of an anhydrous mixture of methylene chloride and methanol 4:1. 176 mg (3.27 mM) of sodium methylate dissolved in 50 ml of anhydrous methanol are added and the mixture is refluxed for 2 hours.

After the reaction, the mixture is neutralized with Dowex AG 50x8 anhydrous resin in the $H^+$ form. The resin is separated by filtration, washed with methanol, and the solution evaporated to dryness. The residue is taken up in 50 ml of methylene chloride/methanol 1:1 and the reaction product is precipitated by pouring it into 250 ml of acetone. The raw product (4.9 g) is purified by preparative high pressure chromatography on silica gel, 60 H Merck, using as solvent a mixture of chloroform/methanol/isopropanol/ammonium carbonate (2%) 1140:820:180:140. The pure fractions are pooled, evaporated to dryness, redissolved in 15 ml of chloroform/methanol 1:1, and the product is precipitated with 75 ml of acetone. This represents the methyl ester of $GM_1$ ganglioside. Yield 4.2 g.

IR spectroscopy on KBr pellets shows the typical ester bond at 1750 $cm^{-1}$.

Silica gel chromatography with chloroform/methanol/$CaCl_2$ (0.3%) 55:45:10 and determination with Ehrlich reagent (Rf 0.72) shows the product to be a unitary compound free from the inner ester used as the starting product (Rf 0.75), and free from $GM_2$ ganglioside (Rf 0.65). Treatment with a 0.1N solution of $Na_2CO_3$ at 60°C for one hour splits the ester bond and yields the primary product, $GM_1$.

## EXAMPLE 4

### Pharmaceutical preparations in solution for injection

| Preparation No. 1 - one 2-ml ampule contains: | |
|---|---|
| GM$_1$ methyl ester | mg 5 |
| sodium chloride | mg 16 |
| citrate buffer pH 6 in water for injection q.s. ad | ml 2 |

| Preparation No. 2 - one 2-ml ampule contains: | |
|---|---|
| GM$_1$ methyl ester | mg 50 |
| sodium chloride | mg 16 |
| citrate buffer pH 6 in water for injection q.s. ad | ml 2 |

| Preparation No. 3 - one 4-ml vial contains: | |
|---|---|
| GM$_1$ methyl ester | mg 100 |
| sodium chloride | mg 32 |
| citrate buffer pH 6 in water for injection q.s. ad | ml 4 |

EXAMPLE 5

Pharmaceutical compositions prepared in twin vials

The preparations illustrated in this Example are presented in sets of two vials. The first contains the active substance in the form of a freeze-dried powder, in quantities varying between 10% and 90% in weight, together with a pharmaceutically acceptable excipient, with glycine or mannitol. The second vial contains the solvent, such as a solution of sodium chloride and a citrate buffer.

In the case of systems 4 and 5, the vial containing the active principle can be prepared by freeze-drying, using water or another solvent (such as terbutanol), or by directly filling the vial with powder under aseptic conditions.

System No. 1

a. One 2-ml vial of freeze-dried powder contains:

| GM$_1$ methyl ester | mg 5 |
|---|---|
| glycine | mg 30 |

b. One 2-ml ampule of solvent contains:

| sodium chloride | mg 16 |
|---|---|
| citrate buffer in water for injection q.s. ad | ml 2 |

System No. 2

a. One 5-ml vial of freeze-dried powder contains:

| GM$_1$ methyl ester | mg 150 |
|---|---|
| glycine | mg 50 |

b. One 4-ml ampule of solvent contains:

| sodium chloride | mg 32 |
| citrate buffer in water for injection q.s. ad | ml 4 |

System No. 3

   a. One 3-ml vial of freeze-dried powder contains:

| $GM_1$ methyl ester | mg 50 |
| glycine | mg 25 |

   b. One 3-ml ampule of solvent contains:

| sodium chloride | mg 24 |
| citrate buffer in water for injection q.s. ad | ml 3 |

System No. 4

   a. One vial contains:

| $GM_1$ methyl ester | mg 50 |

   b. One 2-ml ampule of solvent contains:

| monobasic sodium phosphate-$2H_2O$ | mg 0.5 |
| dibasic sodium phosphate-$12H_2O$ | mg 6 |
| mannitol | mg 80 |
| water for injection | ml 2 |

System No. 5

   a. One vial contains:

| $GM_1$ methyl ester | mg 100 |

   b. One 4-ml ampule of solvent contains:

| monobasic sodium phosphate-$2H_2O$ | mg 1 |
| dibasic sodium phosphate-$12H_2O$ | mg 12 |
| mannitol | mg 160 |
| water for injection | ml 4 |

   The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

**Claims**

1. Use of the methyl ester of ganglioside GM$_1$ for the manufacture of a pharmaceutical composition for preventing or treating neuronotoxic damage in a patient.

2. Use according to claim 1, wherein said neuronotoxic damage is caused by the action of at least one excitatory amino acid.

3. Use according to claim 2, wherein wherein said excitatory amino acid is selected from the group consisting of glutamic acid, aspartic acid, N-methyl-D-aspartic acid, $\beta$-N-oxalylamino-L-alanine, alanosine, cysteic acid, cysteine sulfinic acid, homocysteic acid, cysteine-S-sulfonic acid, ibotenic acid, quisqualic acid, kainic acid, domoic acid, and $\beta$-N-methylamino-L-alanine.

4. Use according to any one of claims 1 to 3, wherein said neuronotoxic damage is associated with ischemia, hypoxia, trauma and compression, metabolic dysfunction, epilepsy, neurodegenerative disease, chronic pain, or cerebral infarct.

5. Use according to claim 4, wherein said methyl ester of GM$_1$ ganglioside is administered intramuscularly, subcutaneously, or intradermally in combination with one or more pharmaceutically acceptable excipients, vectors, or diluents.

6. Use according to claim 4, wherein said methyl ester of GM$_1$ ganglioside is administered by intravenous injection or by infusion.

7. A pharmaceutical composition for the prevention or treatment of neuronotoxic damage in a subject, comprising the methyl ester of GM$_1$ ganglioside and a pharmaceutically acceptable excipient, vector, or diluent.

European Patent
Office

EUROPEAN SEARCH REPORT

Application Number

EP    92 10 9069

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X<br>D,Y | EP-A-0 167 449 (FIDIA SPA) 8 January 1986<br>* abstract *<br>* page 82 - page 93; example 1 *<br>--- | 1,4-7<br>2-3 | A61K31/70 |
| Y | CHEMICAL ABSTRACTS, vol. 112,<br>1990, Columbus, Ohio, US;<br>abstract no. 69817V,<br>SKAPER ET AL.: 'MONOSIALOGANGLIOSIDE GM1<br>PROTECTS AGAINST ANOXIA-INDUCED NEURONAL DEATH<br>IN VITRO'<br>page 63 ;<br>* abstract *<br>--- | 2-3 | |
| Y | CHEMICAL ABSTRACTS, vol. 114,<br>1991, Columbus, Ohio, US;<br>abstract no. 56122F,<br>FACCI ET AL.: 'HYPOGLYCEMIC NEUROTOXICITY IN<br>VITRO: INVOLVEMENT OF EXCITATORY AMINO ACID<br>RECEPTORS AND ATTENUATION BY<br>MONOSIALOGANGLIOSIDE GM1'<br>page 108 ;<br>* abstract *<br>--- | 2-3 | TECHNICAL FIELDS<br>SEARCHED (Int. Cl.5 ) |
| D,Y | ANNUAL REVIEW OF PHARMACOLOGY AND TOXICOLOGY<br>vol. 30, 1990,<br>pages 47 - 71;<br>J.W. OLNEY: 'EXCITATORY AMINO ACIDS AND<br>NEUROPSYCHIATRIC DISORDERS'<br>* the whole document *<br>--- | 2-3 | A61K |
| D,Y | NEUROSCIENCE LETTERS<br>vol. 117, 1990,<br>pages 154 - 159;<br>S.D. SKAPER ET AL.: 'GANGLIOSIDES ATTENUATE THE<br>DELAYED NEUROTOXICITY OF ASPARTIC ACID IN VITRO'<br>* the whole document *<br>--- | 2-3 | |
| | -/-- | | |

The present search report has been drawn up for all claims

| Place of search | Date of completion of the search | Examiner |
|---|---|---|
| THE HAGUE | 11 AUGUST 1992 | HOFF P.J.L. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................................
& : member of the same patent family, corresponding
   document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | PROC. NATL. ACAD. SCI. vol. 85, 1988, pages 7351 - 7355; M. FAVARON ET AL.: 'GANGLIOSIDES PREVENT GLUTAMATE AND KAINATE NEUROTOXICITY IN PRIMARY NEURONAL CULTURES OF NEONATAL RAT CEREBELLUM AND CORTEX' * the whole document * | 2-3 | |
| Y | JOURNAL OF NEUROSCIENCE RESEARCH vol. 27, no. 2, 1990, pages 202 - 210; L. FACCI ET AL.: 'EXCITATORY AMINO ACID NEUROTOXICITY IN CULTURED RETINAL NEURONS: INVOLVEMENT OF N-METHYL-D-ASPARTATE (NMDA) AND NON-NMDA RECEPTORS AND EFFECT OF GANGLIOSIDE GM1' * the whole document * | 2-3 | |
| A | BRAIN RESEARCH vol. 513, no. 2, pages 286 - 294; M.S. CANNELLA ET AL.: 'DERIVATIVES OF GANGLIOSIDE GM1 AS NEURONOTROPHIC AGENTS: COMPARISON OF IN VIVO AND IN VITRO EFFECTS' * the whole document * | 1-7 | **TECHNICAL FIELDS SEARCHED (Int. Cl.5)** |

The present search report has been drawn up for all claims

| Place of search | Date of completion of the search | Examiner |
|---|---|---|
| THE HAGUE | 11 AUGUST 1992 | HOFF P.J.L. |

EPO FORM 1503 03.82 (P0401)